# EUROPEAN PATENT APPLICATION

(11) **EP 0 867 723 A2**
(43) Date of publication of application: **30.09.1998**
(21) Application number: 98201760.0
(22) Date of filing: 28.06.1991
(51) Int. Cl.: G01N 33/86

(54) **Fibrinogen assays using dry chemical reagents containing magnetic particles**

(30) Priority: 10.07.1990 US 550570
(62) Divisional of application: 91914171.3
(71) Applicant: CARDIOVASCULAR DIAGNOSTICS INC., Research Triangle Park, NC 27709 (US)
(72) Inventor: Oberhardt, Bruce J., Raleigh, NC 27615 (US); Gresalfi, Nancy, Durham, NC 27705 (US)
(74) Representative: Des Termes, Monique

(57) **Abstract**

A centrifugal separator comprises a separating drum having an opening at at least one end thereof and a vertical axis, revolving means for revolving the separating drum around a vertical line spaced apart by a predetermined distance from the axis of the separating drum, rotating means for rotating the separating drum about its own axis, and feeder means for supplying the fluid to be treated for separation to the separating drum.

## Description

### BACKGROUND OF THE INVENTION

The present invention relates to centrifugal separators, for example, for use in treating sludge sedimented in rivers, lakes and marshes, soiled waters discharged from iron mills and containing iron particles, coal, etc., and muddy water or effluent resulting from civil engineering work to separate the sludge, muddy water or the like into water and semi- hydrated solids, or for use in separating air containing a powder such as cement into the powder and air.

Various apparatus such as filter presses and dehydrators incorporating a centrifugal drum are conventionally employed for these uses.

The conventional apparatus of any type use a filter, which is liable to clog, therefore requires cleaning or replacement in a short period of time and is cumbersome to use.

Further when the filter is used, for example, the water and the semihydrated solid matter as separated from each other must be further treated individually so as not to become mixed again, whereas the semihydrated solid matter, which is no longer fluid, is not easy to treat.

### SUMMARY OF THE INVENTION

An object of the present invention is to provide a centrifugal separator for separating a fluid containing solid matter into a liquid or gas, and solids reliably without necessitating a cumbersome procedure or treatment.

The present invention provides a centrifugal separator comprising a separating drum having an opening at at least one end thereof and a vertical axis, revolving means for revolving the separating drum around a vertical line spaced apart by a predetermined distance from the axis of the separating drum, rotating means for rotating the separating drum about its own axis, and feeder means for supplying the fluid to be treated for separation to the separating drum.

When the fluid to be treated is supplied to the separating drum of the separator of the invention, the fluid is separated by the centrifugal force of revolution into solid matter contained in the fluid and a liquid or gas owing to a difference in specific gravity therebetween, and the solid matter adheres to the inner surface of the separating drum, while the liquid or gas forms a layer over the surface of the solid matter and is discharged from the drum through the opening thereof. In the meantime, the separating drum rotates about its own axis, permitting the solid matter adhering to the drum inner surface to face toward different directions against the centrifugal force. The solid matter is released from the drum inner surface upon the centrifugal force overcoming the adhesion of the solid matter.

According to the present invention, therefore, the fluid containing solids can be reliably separated into the solid matter and a liquid or gas without necessitating a cumbersome procedure or treatment.

Preferably, the separating drum has a peripheral wall formed with an aperture for passing therethrough the fluid to be treated, the rotating means being intermittently controllable so as to stop the separating drum with the aperture facing inward, the feeder means being intermittently controllable so as to supply the fluid to be treated to the separating drum as stopped with the aperture facing inward.

While the aperture is facing inward, the fluid is efficiently separated into the solid matter and the liquid or gas. When the aperture is directed outward, the solid matter is released from the drum inner surface and centrifugally discharged through the aperture.

The feeder means may have a hollow supply member disposed inwardly of the separating drum and revolvable along with the separating drum, the supply member having an outlet positionable as opposed to the inwardly facing aperture.

The fluid to be delivered from the outlet for treatment is forced outward by the centrifugal force of revolution, and the fluid thus forced outward is led into the separating drum without stagnation through the aperture as directed inward. For example, if the feeder means has a bent fluid supply channel, the fluid will be inadvertently separated at the bent portion. It is then likely that the solids separated off will adhere to the channel wall to clog up the channel, whereas such trouble is avoidable when the fluid is forced out straight.

When the separating drum is provided inside thereof with a plurality of baffle plates arranged one above another, an elongated fluid channel can be provided inside the separating drum.

The fluid channel can be further elongated to achieve an improved separation efficiency if the baffle plates are each in the form of a horizontal plate and are so arranged that each of the baffle plates partially laps over another one of the baffle plates immediately adjacent thereto when viewed axially of the separating drum.

When the separating drum is provided with a discharge opening at an upper end thereof and an outflow preventing wall at a lower end thereof, the revolution of the separating drum causes the fluid to flow upward inside the drum.

The separating drum may be provided inside thereof with a partition plate positioned at a higher level than a fluid outlet of the feeder means so as to partially occupy the cross section of the separating drum, so that the fluid supplied to the separating drum by one cycle of operation of the feeder means can be prevented by the partition plate from flowing upward owing to the centrifugal force of revolution of the fluid, with the aperture facing inward. This assures reliable separation of the fluid in the separating drum as held out of rotation about its own axis, with the aperture facing inward.

If the separating drum is temporarily held out of rotation about its own axis in the course of movement of the aperture from the inwardly facing position to an outwardly facing position, the fluid can be separated more effectively before discharge through the aperture.

When the separating drum has its opening covered with a filter, the water to be discharged from the drum through its opening is filtered to ensure more effective separation. The filter will not be clogged since the solids acting to adhere to the filter are moved centrifugally.

### BRIEF DESCRIPTION OF THE DRAWINGS

FIG. 1 is a perspective view partly broken away along a vertical plane and showing a centrifugal separator embodying the invention;
FIG. 2 is a view in vertical section of the same;
FIG. 3 is a perspective view of a separating drum of the same;
FIG. 4 is a view in horizontal cross section of the same;
FIG. 5 is a time chart showing the operation of the separator;
FIGS. 6 (a) and 6 (b) are diagrams for illustrating the operation of the separator;
FIG. 7 is a view in vertical section corresponding to FIG. 2 and showing a separating drum of a modification of the separator;
FIG. 8 is a perspective view of the separating drum;
FIGS. 9 (a) to 9 (d) are diagrams for illustrating the operation of the separating drum;
FIG. 10 is a time chart showing the operation of the separating drum;
FIG. 11 is a view in vertical section corresponding to FIG. 7 and showing a separating drum of another modification of the separator;
FIG. 12 is a perspective view of the separating drum;
FIGS. 13 (a) to 13 (d) are diagrams for illustrating the operation of the separating drum;
FIG. 14 is a diagram for illustrating an operation for preventing a filter of the drum from clogging;
FIG. 15 is an enlarged view in section of the filter;
FIG. 16 is a sectional view corresponding to FIG. 11 and showing two filters as used in the separating drum; and

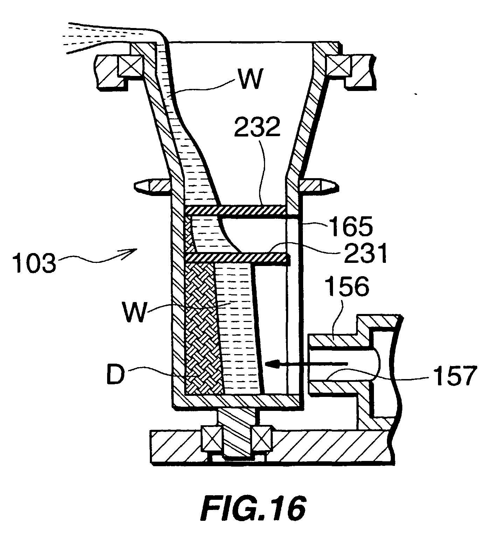

FIG. 16 is a time chart showing the operation of the separating drum.

### DESCRIPTION OF THE PREFERRED EMBODIMENTS

Embodiments of the invention will be described below with reference to the drawings.

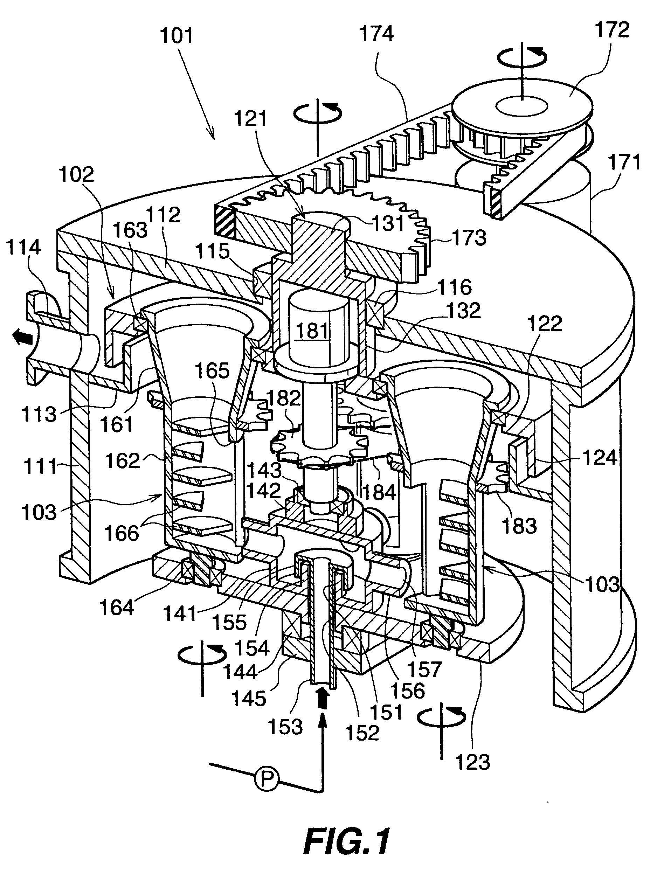

Referring to FIG. 1 showing a centrifugal separator, the separator comprises a hollow body 101, a rotary assembly 102 accommodated in the body 101 and four separating drums 103 each in the form of a vertical hollow cylinder and mounted on the rotary assembly 102.

The body 101 comprises a vertical tubular trunk wall 111 and a top wall 112 in the form of a flat plate and covering an upper-end opening thereof.

The trunk wall 111 is provided with a trough 113 positioned inside thereof slightly above the midportion of its height and a discharge pipe 114 in communication with the trough 113. A support bar 145 in the form of a horizontal strip extends across a lower-end opening of the trunk wall 111 through the trunk wall axis and is attached to the lower end of the trunk wall 111. A lower main bearing 144 is mounted on the upper side of the support bar 145 centrally thereof and coaxially with the trunk wall.

A main motor 171 facing vertically upward is mounted on the trunk wall outside thereof close to its upper end. A main drive sprocket 172 is fixed to the output shaft of the main motor 171.

The top wall 112 is formed in its central portion with a shaft hole 115 having an upper main bearing 116 fitted therein.

The rotary assembly 101 comprises a rotary shaft 121 inserted through the bearing 116, a top wall 122 secured directly to the lower end of the shaft 121, and a bottom wall 123 disposed below the top wall 122 at a distance therefrom and connected to the top wall 122 by the separating drums 103. A skirt 124 extending into the trough 113 is integral with the outer periphery of the top wall 122.

The rotary shaft 121 comprises an upper solid shaft portion 131 having a small diameter and a lower hollow shaft portion 132 having a large diameter. The solid shaft portion 131 projects upward beyond the upper main bearing 116 and has a main driven sprocket 173 fixed to the projection. The sprocket 173 is coupled to the main drive sprocket 172 by a belt 174. The hollow shaft portion 132 is supported by the bearing 116.

Housed in the hollow shaft portion 132 is a secondary motor 181 fixed to the top wall 122 of the rotary assembly. The rotary shaft of the motor 181 extends through the top wall 122 to project downward and is supported by a secondary bearing 143. A secondary drive sprocket 182 is fixed to the rotary shaft of the secondary motor 181.

A distribution chamber 141 is fixedly provided on the upper side of the bottom wall 123 centrally thereof. The secondary bearing 143 is attached to the upper side of top wall of the chamber 141 with a support member 142 interposed therebetween. The bottom plate 123 is supported at the center of its lower side by the lower main bearing 144.

A bottom wall of the distribution chamber 141 and the bottom wall of the rotary assembly 101 are formed respectively with upper and lower pipe holes 151, 152 in register, and a feed pipe 153 for the water to be treated is inserted through these holes. The bottom wall inner periphery defining the upper pipe hole 151 is provided with a vertical sleeve 154. The feed pipe 153 is provided at its upper end with a seal ring 155 inverted L-shaped in cross section and covering the sleeve 154. The chamber 141 has a peripheral wall formed with four supply pipes 156 arranged radially at equal spacings. The four supply pipes 156 extend toward the axes of the respective four separating drums 103 and each have an outlet 157 at the outer end.

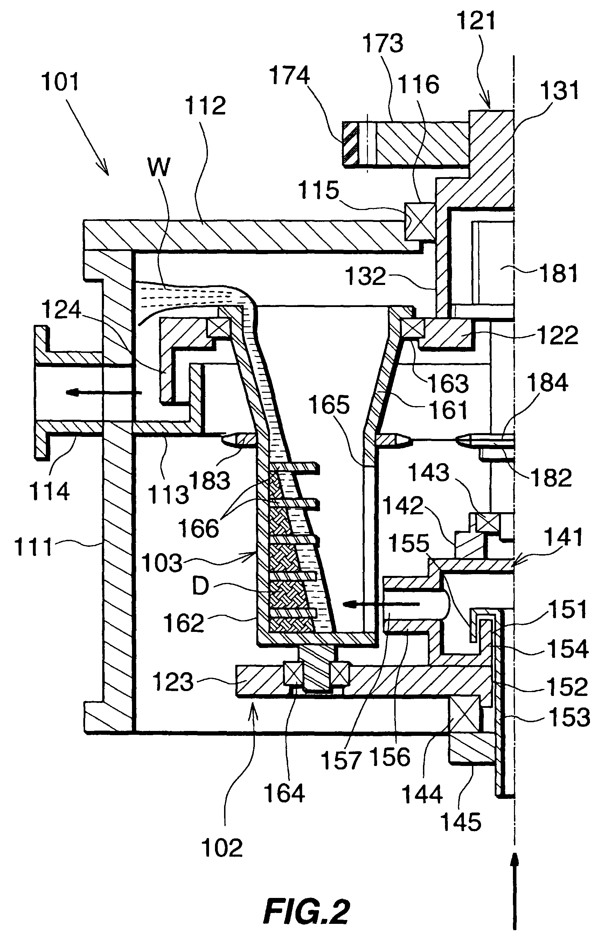

As shown in greater detail in FIG. 2, the separating drum 103 comprises an upwardly flaring portion 161 and a lower straight portion 162. The flaring portion 161 is supported by a bearing 163 on the rotary assembly top wall 122, and the straight portion 162 by a bearing 164 on the rotary assembly bottom wall 123. The straight portion 132 is formed with a vertically elongated aperture 165 for passing therethrough the fluid to be treated. When facing inward, the aperture 165 is opposed to the outlet 157 of the supply pipe 156.

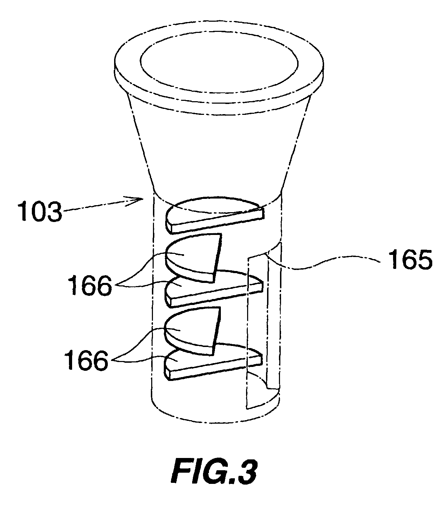

The straight portion 162 has a plurality of baffle plates 166 arranged one above another. As shown in greater detail in FIG. 3, the baffle plates 166 are each in the form of a horizontal semicircle. Each baffle plate 166 overlaps another adjacent plate 166 immediately therebelow by a 2/3 portion of the entire area.

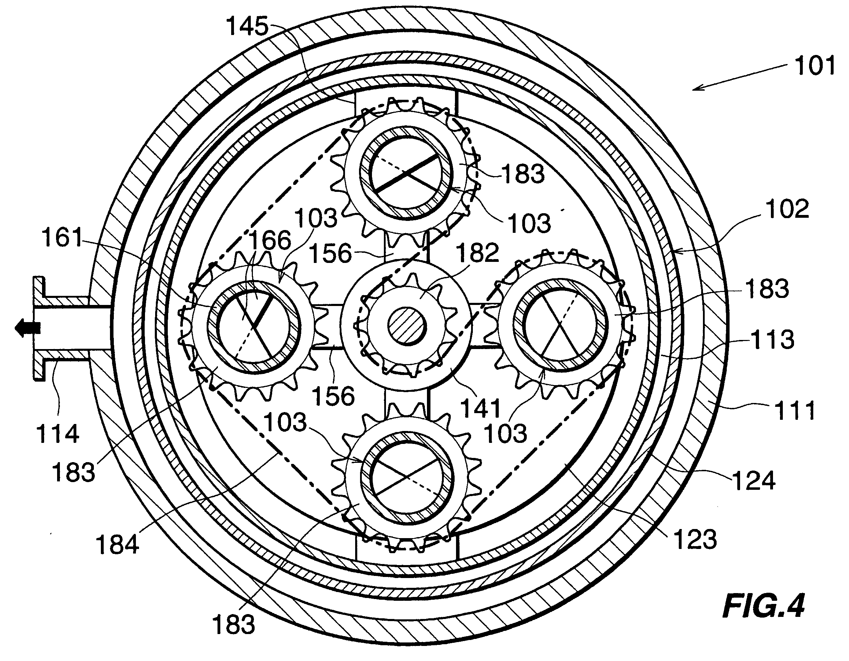

A secondary driven sprocket 183 is secured to the lower end of the flaring portion 161 on the outer side thereof. As seen in FIG. 4, a chain 184 is reeved around the secondary drive sprocket 182 and the secondary driven sprocket 183.

When the main motor 171 is operated, the rotary assembly 101 rotates about its shaft 121, revolving the separating drums 103 around the axis of the shaft 121. On the other hand, the secondary motor 181, when operated, moves the chain 184, whereby the four separating drums 103 are rotated at the same time each about its own axis.

The speed of revolution of the separating drums 103 is suitably determined according to the properties of the water to be separated. The drums 103 are revolved, for example, at a peripheral speed of 500 to 1000 m/min. When the radius of gyration of the revolution is about 400 mm, the speed of revolution is 600 rpm.

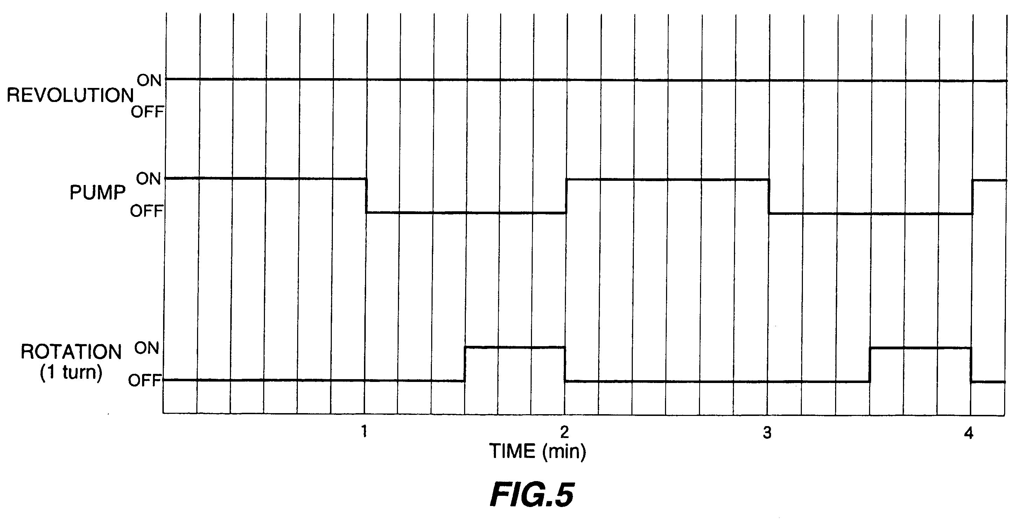

As shown in FIG. 5, the separating drums 103 are revolved continuously. In the meantime, a pump is operated intermittently to supply the water to be separated intermittently, for example, in such manner that the water is supplied for 1 minute, followed by a cessation of supply for 1 minute. During the supply of water, the separating drums 103 are stopped from rotating about their axes, with the apertures 165 facing inward with respect to the radius of gyration of the revolution. During the cessation of supply of the water, the separating drums 103 are rotated once about their axes, whereby the apertures 165 facing inward are turned outward and thereafter directed inward again. The separating drums 103 may be initiated into rotation about their axes simultaneously with the discontinuation of supply of the water or upon lapse of a predetermined period of time thereafter. The separating drums 103 may be rotated about their axes continuously through 360 degrees, or may be rotated through 180 degrees, then stopped for a predetermined period of time and thereafter rotated in the opposite direction through 180 degrees.

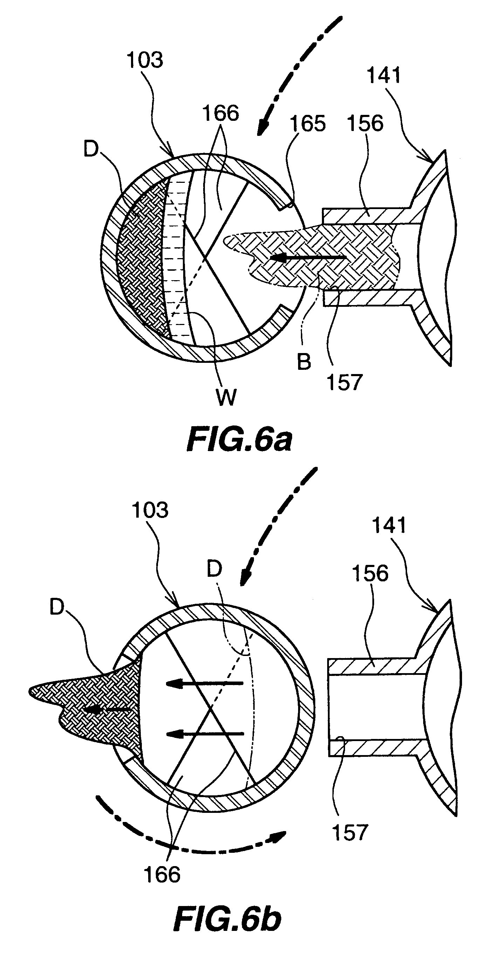

When the water B to be treated is supplied to the distribution chamber 141 through the feed pipe 153 with the aperture 165 of each separating drum 103 opposed to the outlet 157 of the corresponding supply pipe 156 as seen in FIG. 6 (a), the water B supplied is centrifugally forced toward the peripheral wall of the chamber 141 by virtue of rotation of the rotary assembly 102 and discharged through the pipe 156 from the outlet 157 thereof. The water B discharged from the outlet 157 is further centrifugally accelerated, forced outward straight and flows into the separating drum 103 through the aperture 165. Being forced outward straight, the water B is unlikely to spill through a small clearance which may be formed between the edge of the drum 103 defining the aperture 165 and the edge of outlet 157 of the supply pipe 156.

When a predetermined period of time elapses after the water B has been introduced into the separating drum 103, the water B is separated into solid matter D and water W, and the water W is discharged from the upper-end opening of the drum 103. When the drum 103 is rotated through 180 degrees as shown in FIG. 6 (b), the solid matter D separated off is discharged from the drum 103 through the aperture 165.

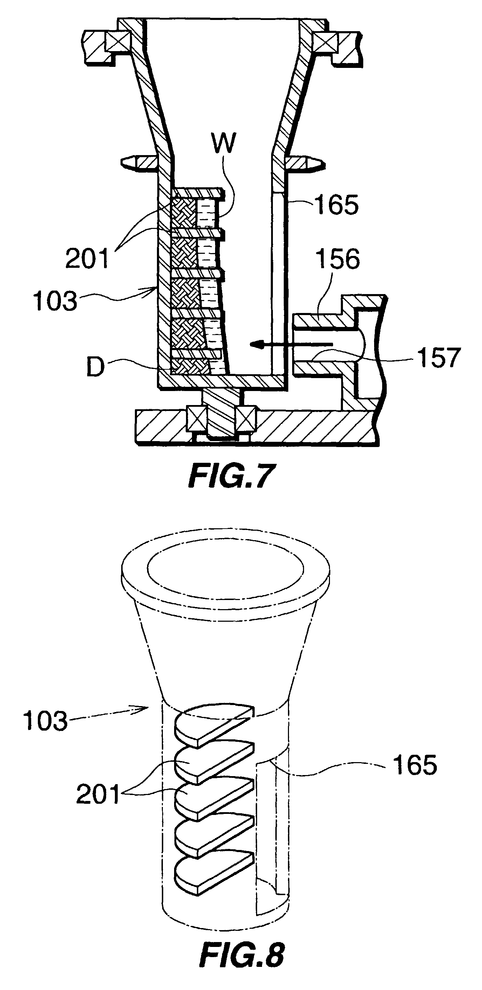

A modification of the centrifugal separator will be further described with reference to FIGS. 7 to 10. The modification basically has the same construction as the centrifugal separator shown in FIGS. 1 to 6.

FIGS. 7 and 8 show a separating drum 103 having a plurality of partition plates 201 arranged one above another inside the drum. The partition plates 201 have the same shape as the baffle plates 166 and are each in the form of a horizontal semicircle. All the partition plates 201 are arranged wholly in register. When an aperture 165 for passing therethrough the water to be treated is positioned as directed inward and when the drum 103 is seen in cross section, the partition plate 201 occupies the semicircular portion of the drum 103 opposite to the aperture 165, and the straight end of the partition plate 201 opposed to the aperture 165 is orthogonal to the aperture 165.

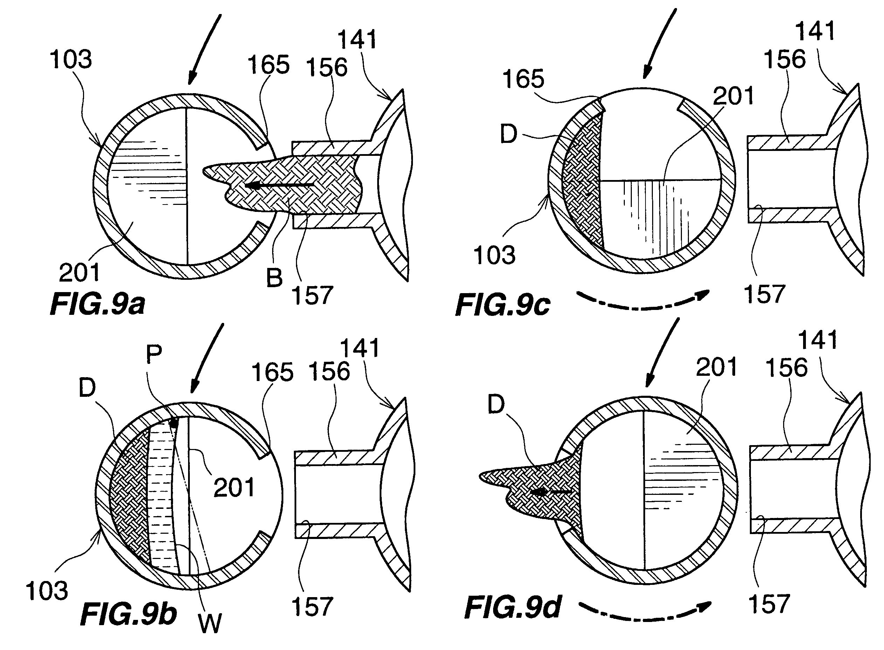

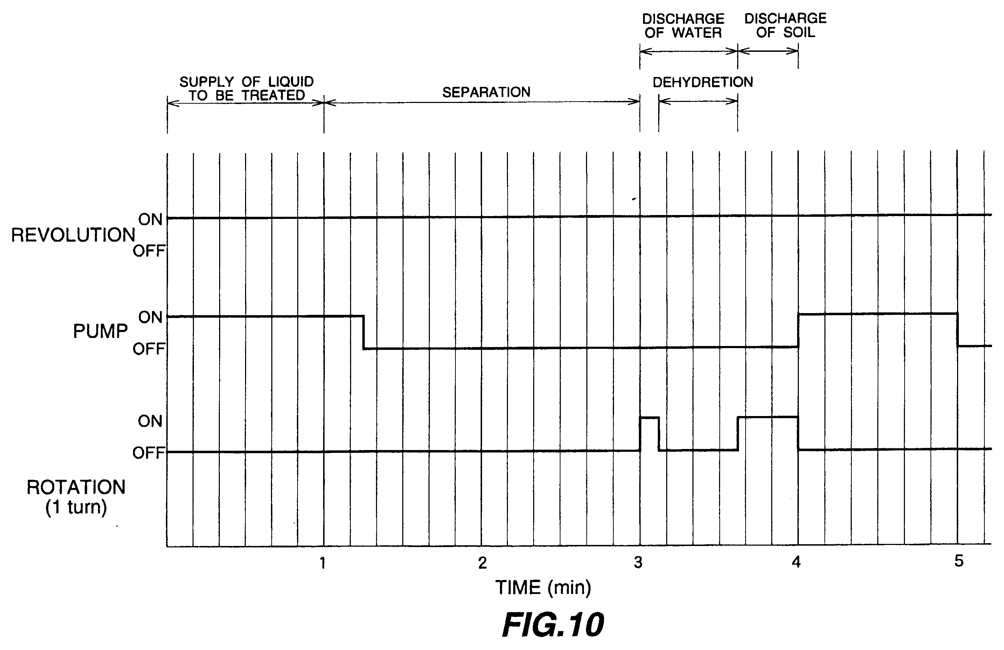

The operation of the separator will be described next with reference to FIGS. 9 and 10.

As shown in FIG. 9 (a), the water B to be treated is supplied to the separating drum 103 as held out of rotation about its own axis, with the aperture 165 facing inward. The water B is supplied approximately at the level of the lowermost partition plate 201. The water B supplied is caused to flow upward over the lowermost partition plate 201 toward another plate 201 thereabove within the drum 103 by the centrifugal force of revolution of the drum, thus passing over the partition plates 201 one after another. However, the amount of the water B to be supplied during one cycle of operation is so determined that the water will not pass over the uppermost partition plate 201 (see FIG. 7). Thus, the quantity of water supplied by one cycle of operation is collected below the uppermost partition plate 201.

The water B collected below the uppermost partition plate 201 is completely separated into solid matter D and water W while the separating drum 103 is held at rest before the start of rotation about its own axis as seen in FIG. 7.

With the start of rotation of the drum 103, the partition plates 201 are also rotated with the drum 103. When the straight ends of partition plates 201 become submerged in the separated water W at an angle with respect to the inward or outward direction as indicated in a phantom line in FIG. 9 (b), the separated water W starts to flow upward at a point P indicated by a solid spot mark in the drawing, passing over the partition plates 201 one after another, and is discharged from the upper end of the drum 103.

The drum 103 is rotated through 90 degrees as shown in FIG. 9 (c) and then temporarily held out of rotation. Although the rotation takes a relatively short period of time, the solid matter D and the water W separated from each other differ in adhesion or fluidity and therefore behave differently. The water W separated off moves with the rotation of the drum 103 owing to the centrifugal force acting thereon, shifting every moment, and is discharged from the drum 103, whereas the solid matter D tends to remain in position, and therefore remains inside the drum 103, adhering to the inner surface of the drum 103.

With the separating drum 103 held out of rotation after rotating through 90 degrees, the solid matter D is subjected to the centrifugal force, whereby the solid matter D is further separated, and the water W separated off is discharged from the upper end of the drum 103.

Upon lapse of a predetermined period of time after the drum 103 has been rotated through 90 degrees, the drum 103 resumes rotation about its own axis. When the drum 103 is rotated further through 90 degrees through the resumed rotation as shown in FIG. 9 (d), the aperture 165 faces outward, and the solid matter D centrifugally acted on is discharged through the aperture 165 thus facing outward.

The rotation of the separating drum 103 may be discontinued when the aperture 165 is brought to the outwardly facing position, but the drum need not be so stopped since the solid matter D is discharged rapidly. When the drum 103 is continuously further rotated about its own axis, moving the aperture 165 from the outwardly facing position to the inwardly facing position through 180 degrees, the drum 103 finishes one cycle of operation. The separating drum 103 may be rotated reversely every time it has been rotated through 180 degrees instead of being rotated through 360 degrees every cycle.

The present embodiment is so adapted that the water to be treated in the drum is separated as it is wholly collected therein and held at rest. In this respect, the embodiment distinctly differs from the first embodiment. With the first embodiment, the water supplied to the separating drum is subjected to a separating treatment while allowing the water separated off to gradually flow out from the drum upper end at the same time. When the water to be treated is thus moved while being centrifugally acted on, solids are likely to be entrained in the moving water to result in a lower separation efficiency. Further if an increased centrifugal force is used to increase the separation capacity, the water separated off is more likely to entrain solids therein, such that an increased centrifugal force fails to improve the separation capacity greatly as expected. Since the water to be treated is held at rest within the separating drum during separation according to the present embodiment, an increased separation capacity is expectable.

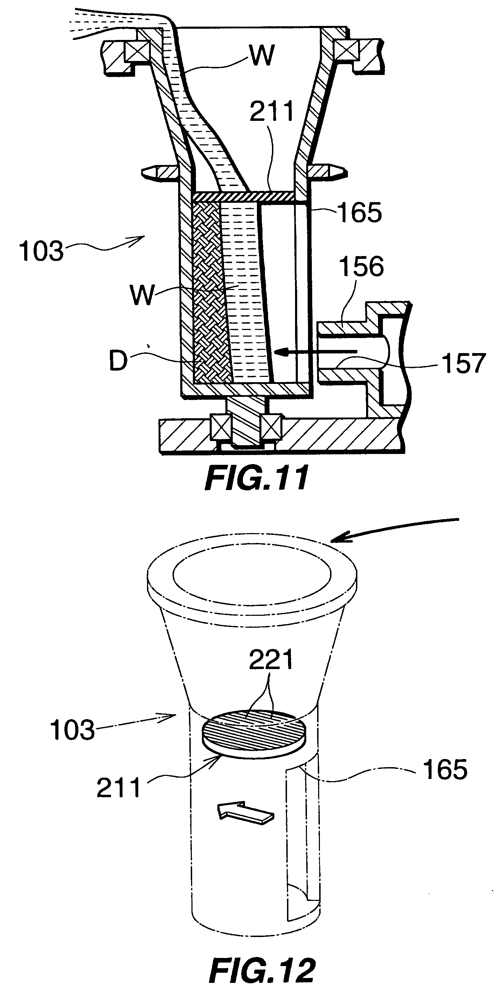

Another modification of the centrifugal separator will be described with reference to FIGS. 11 to 17.

Referring to FIG. 11, a filter 211 is used in place of the partition plates 201 provided inside the separating drum 103 of FIG. 7. The filter 211 is positioned immediately above an aperture 165 for passing the fluid to be treated, and is in the form of a horizontal disk covering the entire upper-end opening of a separating drum 103.

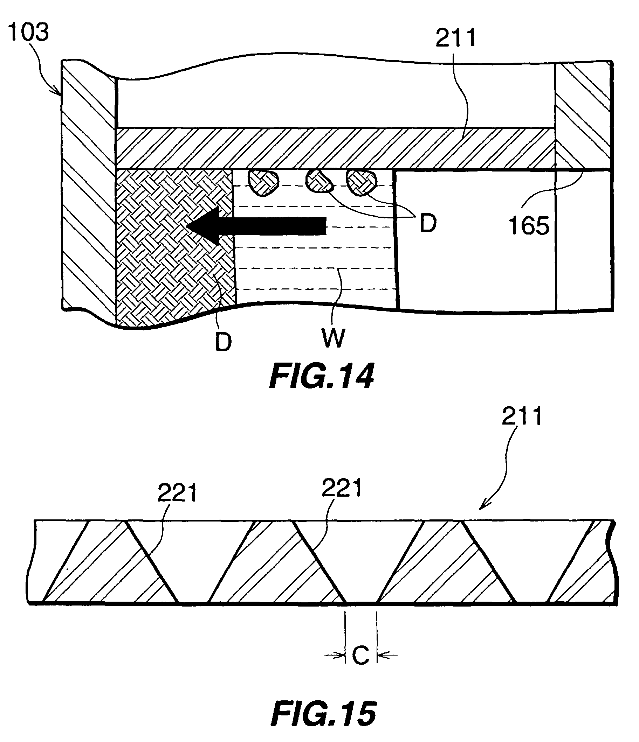

As shown in FIG. 12, the filter is made of a so-called wedge wire and has parallel passageways 221. With the aperture 165 facing toward the center of revolution, the lengthwise direction of the passageways 221 is positioned radially of the path of revolution. As shown in greater detail in FIG. 15, the passageways 221 are wedge-shaped in cross section and are about 30 micrometers in minimum width C at the lower ends thereof.

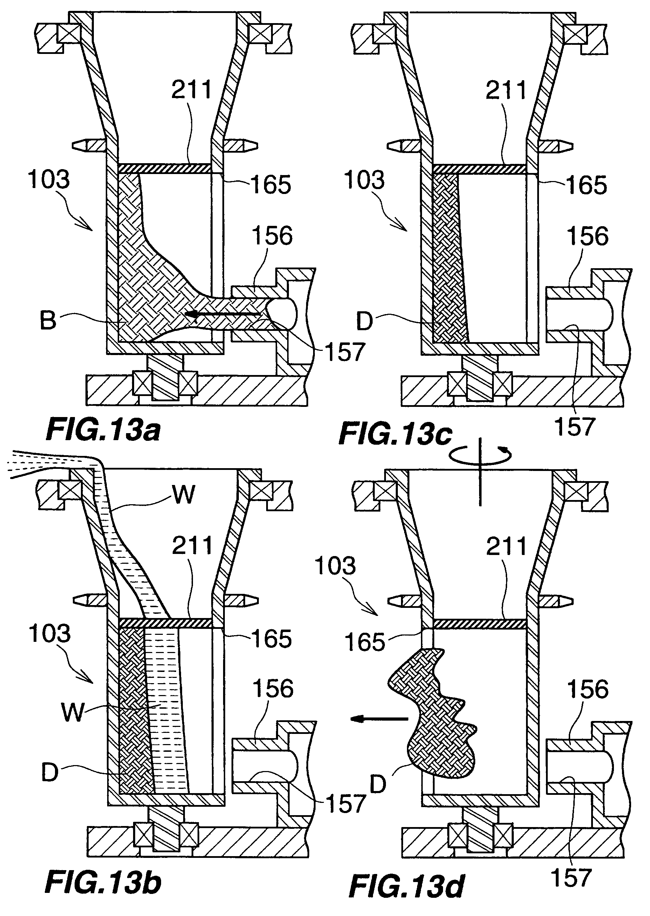

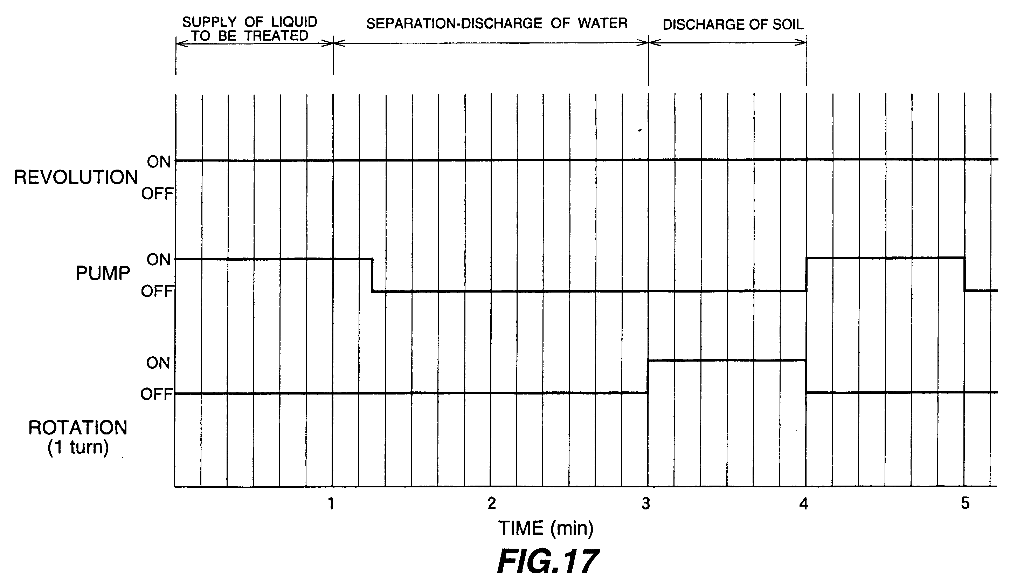

When the water to be treated is supplied to the separating drum 103 through the outlet 157 of the supply pipe 156 with the aperture 165 facing inward while the drum 103 is being revolved [FIG. 13 (a)], the water supplied is collected under the filter 211. The water collected under the filter 211 is then separated into water and solid matter by the centrifugal force of the revolution, and the water separated off passes through the filter 211 and is discharged from the drum 103 through its upper-end opening [FIG. 13 (b)]. On the other hand, the solid matter remains collected under the filter 211 without passing therethrough [FIG. 13 (c)]. After the water separated off has been discharged from the drum 103, the drum 103 is rotated about its own axis, whereby the aperture 165 is brought from the inwardly facing position to an outwardly facing position. Whereas the separating drum 103 is halted during rotation about its own axis in the case of the embodiment shown in FIGS. 7 to 10, the drum 103 of the present embodiment need not be so stopped. When the aperture 165 is brought to the outwardly facing position, the solid matter is released from the drum inner surface by the centrifugal force of the revolution and delivered from the separating drum 103 through the aperture 165 [FIG. 13 (d)]. The water to be treated is thereafter supplied to the drum 103 when the aperture 165 is brought to the inwardly facing position again. FIG. 17 is a time chart showing the separating operation described above.

Initially when the separating operation is started, solids adhere to the entire area of the portion of the filter 211 in contact with the water to be treated and also to the portion of the filter 211 serving to pass the separated water therethrough as shown in detail in FIG. 14, whereas the adhering solids are forced outwardly of the water passing portion and unlikely to close the water passing portion, with the result that the water separated off smoothly flows through this portion.

FIG. 16 shows a modification wherein two filers are used as positioned one above the other. The openings or passageways of the two filters 231, 232 are so determined that the lower filter 231 serves for rough filtration, and the upper filter 232 for finishing filtration.

Instead of providing two filters in one separating drum, two centrifugal separators may alternatively used. In this case, a filter for rough filtration may be used in each separating drum of the upstream separator, with a filter for finishing filtration used in the downstream separator.

To be effective, the filter is about 5 to about 50 micrometer in opening size. Besides the wedge wire mentioned above, metal netting, fabric, nylon fiber, ceramics, etc. are useful for making the filter.

The present modification may also have baffle plates or partition plates used in the foregoing modification.

While liquid separating operations have been described above, the basic principle of the present invention is applicable also to the separation of gases. In this case, some alternations will be necessary in the design of the centrifugal separator described in accordance with the differences between the liquid and the gas in properties. Such alterations include, for example, gas transport means, seal means for the gas, etc.

## Claims

1. A centrifugal separator comprising:
a separating drum having an opening at at least one end thereof and a vertical axis,
revolving means for revolving the separating drum around a vertical line spaced apart by a predetermined distance from the axis of the separating drum,
rotating means for rotating the separating drum about its own axis, and
feeder means for supplying the fluid to be treated for separation to the separating drum.

2. A centrifugal separator according to claim 1 wherein the separating drum has a peripheral wall formed with an aperture for passing therethrough the fluid to be treated, the rotating means being intermittently controllable so as to stop the separating drum with the aperture facing inward, the feeder means being intermittently controllable so as to supply the fluid to be treated to the separating drum as stopped with the aperture facing inward.

3. A centrifugal separator according to claim 2 wherein the feeder means has a hollow supply member disposed inwardly of the separating drum and revolvable along with the separating drum, the supply member having an outlet positionable as opposed to the inwardly facing aperture.

4. A centrifugal separator according to any one of claims 1 to 3 wherein the separating drum is provided inside thereof with a plurality of baffle plates arranged one above another.

5. A centrifugal separator according to claim 4 wherein the baffle plates are each in the form of a horizontal plate and are so arranged that each of the baffle plates partially laps over another one of the baffle plates immediately adjacent thereto when viewed axially of the separating drum.

6. A centrifugal separator according to any one of claims 1 to 3 wherein the separating drum is provided with a discharge opening at an upper end thereof and an outflow preventing wall at a lower end thereof.

7. A centrifugal separator according to claim 6 wherein the separating drum is provided inside thereof with a partition plate positioned at a higher level than a fluid outlet of the feeder means so as to partially occupy the cross section of the separating drum, so that the fluid supplied to the separating drum by one cycle of operation of the feeder means can be prevented by the partition plate from flowing upward owing to the centrifugal force of revolution of the fluid, with the aperture facing inward.

8. A centrifugal separator according to claim 7 wherein the separating drum is temporarily held out of rotation about its own axis in the course of movement of the aperture from the inwardly facing position to an outwardly facing position.

9. A centrifugal separator according to any one of claims 1 to 3 wherein the separating drum has its opening covered with a filter.

10. A centrifugal separator according to claim 9 wherein the separating drum is provided inside thereof with a plurality of baffle plates arranged one above another.

11. A centrifugal separator according to claim 10 wherein the baffle plates are each in the form of a horizontal plate and are so arranged that each of the baffle plates partially laps over another one of the baffle plates immediately adjacent thereto when viewed axially of the separating drum.

12. A centrifugal separator according to claim 9 wherein the separating drum is provided with a discharge opening at an upper end thereof and an outflow preventing wall at a lower end thereof.

13. A centrifugal separator according to claim 12 wherein the separating drum is provided inside thereof with a partition plate positioned at a higher level than a fluid outlet of the feeder means so as to partially occupy the cross section of the separating drum, so that the fluid supplied to the separating drum by one cycle of operation of the feeder means can be prevented by the partition plate from flowing upward owing to the centrifugal force of revolution of the fluid, with the aperture facing inward.

14. A centrifugal separator according to claim 13 wherein the separating drum is temporarily held out of rotation about its own axis in the course of movement of the aperture from the inwardly facing position to an outwardly facing position.
